(19) Europäisches Patentamt
European Patent Office
Office européen des brevets

(11) **EP 3 134 848 B1**

(12) **EUROPEAN PATENT SPECIFICATION**

(45) Date of publication and mention
of the grant of the patent:
**04.09.2019 Bulletin 2019/36**

(21) Application number: **15722049.2**

(22) Date of filing: **30.03.2015**

(51) Int Cl.:
*G06K 9/00* (2006.01)     *G01N 21/25* (2006.01)
*G06T 7/00* (2017.01)     *G01N 21/17* (2006.01)
*G01N 21/47* (2006.01)

(86) International application number:
**PCT/EP2015/000679**

(87) International publication number:
**WO 2015/161914 (29.10.2015 Gazette 2015/43)**

(54) **METHOD FOR DETECTING MICRO-COLONIES GROWING ON A MEMBRANE OR AN AGAROSE MEDIUM OF A SAMPLE AND A STERILITY TESTING APPARATUS**

VERFAHREN ZUR DETEKTION VON MIKROKOLONIEN, DIE AUF EINER MEMBRAN ODER EINEM AGAROSEMEDIUM EINER PROBE WACHSEN, UND EINE STERILITÄTSÜBERPRÜFUNGSVORRICHTUNG

PROCÉDÉ DE DÉTECTION DE MICRO-COLONIES SE FORMANT SUR UNE MEMBRANE OU UN MILIEU D'AGAROSE D'UN ÉCHANTILLON ET UN APPAREIL DE CONTRÔLE DE STÉRILITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.04.2014 EP 14290114**

(43) Date of publication of application:
**01.03.2017 Bulletin 2017/09**

(73) Proprietor: **Merck Patent GmbH**
**64293 Darmstadt (DE)**

(72) Inventors:
• **FELDEN, Luc**
**68280 Andolsheim (FR)**
• **BOUTHILLON, Marine**
**91300 Massy (FR)**
• **OLIVIER, Stéphane**
**67560 Rosheim (FR)**
• **WOEHL, Pierre**
**67000 Strasbourg (FR)**
• **GUEDON, Pierre**
**83560 Vinon-sur-verdon (FR)**
• **ALLARD, Florian**
**67100 Strasbourg (FR)**

(74) Representative: **Henkel, Breuer & Partner**
**Patentanwälte**
**Maximiliansplatz 21**
**80333 München (DE)**

(56) References cited:
WO-A1-2008/137746    WO-A1-2014/067907
WO-A2-03/022999     WO-A2-2012/072467
WO-A2-2012/119114    US-A- 3 736 432

• **MARCOUX PIERRE R ET AL: "Optical forward-scattering for identification of bacteria within microcolonies", APPLIED MICROBIOLOGY AND BIOTECHNOLOGY, SPRINGER, DE, vol. 98, no. 5, 12 January 2014 (2014-01-12), pages 2243-2254, XP035328487, ISSN: 0175-7598, DOI: 10.1007/S00253-013-5495-4 [retrieved on 2014-01-12]**
• **Kirstin Petersen ET AL: "3D Tracking of Building Processes in Macrotermes", Visual observation and analysis of animal and insect behavior 2012, 11 November 2012 (2012-11-11), pages 1-4, XP055209510, Retrieved from the Internet: URL:http://people.seas.harvard.edu/~jkwerfel/vaib_icpr.pdf [retrieved on 2015-08-25]**

• **PIN-TZU SU ET AL: "Bacterial Colony from Two-Dimensional Division to Three-Dimensional Development", PLOS ONE, vol. 7, no. 11, 14 November 2012 (2012-11-14), page e48098, XP055204761, DOI: 10.1371/journal.pone.0048098**

**Description**

The present invention is directed to a method for detecting micro-colonies on a membrane or an agarose medium.

Current compendial methods for sterility testing in the pharmaceutical industry remain culture-based and include an incubation period of 14 days.

Adenosine triphosphate (ATP) bioluminescence is a well-established rapid method utilising a specific substrate and enzyme combination, luciferin/luciferase, to break down microbial ATP from growing cells and produce visible light, which can be measured using a luminometer. Several commercial systems have been developed for a range of pharmaceutical test applications, including sterility testing, especially for filterable samples where non-microbial ATP in the sample is less of a concern. The test time can be reduced considerably because detection of microbial growth in culture media is accomplished by ATP-bioluminescence, rather than by visible turbidity. Typically, results equivalent to those of compendial tests are available within 7 days or less.

The Milliflex® Rapid Microbiology Detection and Enumeration system from Merck Millipore also uses ATP- bioluminescence to detect microbial cells and is designed specifically for monitoring microbial contamination in filterable samples. It is automated, employing image analysis technology to detect micro-colonies growing directly on the surface of a membrane filter after the addition of bioluminescence reagents. The system is designed to be quantitative, but a method has been developed and validated to use it for a rapid sterility test.

Colorimetric growth detection methods rely on a color change being produced in a growth medium as a result of microbial metabolism during growth, often as a result of $CO_2$ production. An example of a commercial colorimetric assay system, which can be used for sterility testing is the BacT/ALERT® 3D Dual-T Microbial Detection System from bioMerieux. The system is automated and employs sensitive color detection and analysis technology. It can detect both aerobic and anaerobic bacteria, as well as yeasts and molds.

All living cells produce a small amount of fluorescence (auto-fluorescence) and this can be used to detect micro-colonies growing on a solid surface long before they are visible to the naked eye. This technique is particularly useful for filterable samples, where a membrane filter can be incubated on a conventional nutrient medium and scanned using highly sensitive imaging systems to detect micro-colonies, sometimes several days earlier than using traditional colony counting methods.

Autofluorescence detection has been commercialized by Rapid Micro Biosystems as Growth Direct, which uses a large area CCD imaging system without magnification to detect developing micro-colonies. Although not yet validated for testing sterile products, "proof of concept" has been established. The system is automated and employs sensitive color detection and analysis technology. It can detect both aerobic and anaerobic bacteria, as well as yeasts and molds.

On the other hand, cytometry does not rely on microbial growth to detect contamination, but instead uses cell labelling techniques to detect viable microorganisms. This approach has the potential to detect a wide range of organisms, including yeasts and molds, within minutes. Commercial systems utilize combined fluorescent cell labelling and flow cytometry or solid phase cytometry to detect viable microbial cells. Typically, the cells are labelled using a fluorescent dye or a non-fluorescent substrate, which is converted to a fluorochrome in viable cells. Detection of the labelled cells occurs by laser scanning in either a flow cell (flow cytometry), or on a solid phase platform such as a membrane filter (solid phase cytometry). AES Chemunex has developed solid phase cytometry detection systems.

WO 2011/125033 A1 (PCT/IB2011/051481) discloses a method for detecting clusters of biological particles on a membrane or an agarose medium. The method of this state of the art document discloses determining a topographical representation of the surface and detecting on the topographical representation at least one contour defining a region that is likely to correspond to a cluster of biological particles, i.e. a micro-colony.

The method aims to detect micro-colonies at an early stage in their growth, when they are of a diameter that is too small for them to be visible to the naked eye (diameter of a few tenths of micrometers, e.g. 30 μm or less).

For obtaining the topographical representation of the surface the membrane is placed in an open petri dish and scanned while being exposed to the environment. This might lead to additional contamination of the membrane.

On the other hand, EP 1428018 B1 discloses a method for numeration of living cells by detecting micro-colonies derived from in situ cell division using large area imaging. Microbial enumeration tests based on this document include non-destructive aseptic methods for detecting cellular micro-colonies without labeling reagents. These methods allow for the generation of pure cultures which can be used for microbial identification and determination of antimicrobial resistance.

The company COPAN has presented in April 2011 a device for determining the growth of micro-colonies under the trademark WASPLab. An image of a membrane is taken by a plan view photography as well as laser topographic mapping. Then, the data are obtained while the membrane is exposed to the environment. The method is therefore not suitable for sterility testing.

All the above methods either relay on a detection in an open device, the use of additional reagents or are to slow to comply with nowadays requirements for sterility testing.

Recently, WO 2014/005669 A has disclosed a sample preparation device, preferably for sterility testing, comprising a manifold including one or more receptacles for filtration units, preferably one or more additional receptacles for media containers/vials, and at least one inlet and/or outlet port. The receptacle(s) is/are respectively provided with one or more connectors for establishing a fluid connection with mating ports of the filtration units and media containers/vials upon insertion of the same into the respective receptacles. The connectors are in fluid communication with the inlet and outlet port(s) via channels defined in the manifold to allow a desired fluid transfer through the manifold. The filtration units comprise a base part that defines a membrane support, a removable lid for defining a membrane chamber with the base part and sealing the membrane chamber from the environment, and at least one inlet port and at least one outlet port respectively accessible from outside of the filtration unit and communicating with the membrane chamber at positions upstream and downstream of a membrane when the same is provided on the membrane support. The inlet and outlet port(s) are respectively provided with a sealing mechanism formed so as to be opened upon connection with a mating connector on an external receptacle, preferably the connector of the manifold of the sample preparation device, and so as to be automatically re-sealable upon disconnection.

WO2008/137746A1, WO3/022999 A2, MARCOUX PIERRE R ET AL: "Optical forward-scattering for identification of bacteria within microcolonies", APPLIED MICROBIOLOGY AND BIOTECHNOLOGY, 12. January 2014, pages 2043-2054, as well as Kirstin Petersen ET AL: "3D Tracking of Building Processes in Macrotermes", Visual observation and analysis of animal and insect behaviour 2012, 11 November 2012, pages 1-4 (http://people.seas.harvard.edu/~jkwerfel/vaib_icpr.pdf) and PIN-TZU SU ET AL "Bacterial Colony from Two-Dimensional Division to Three-Dimensional Development", PLOS ONE, vol. 7, no. 11, 14 November 2012, disclose a method for detecting microcolonies growing on a membrane comprising the steps of irradiating the sample with light, imaging an incident area of the light and detecting the reflected light scattered or diffused from the membrane.

Further state of the art is disclosed in WO2014/067907 A1, WO2012/119114 A2 and WO2012/072467 A2.

The object of the present invention is to provide a new method and a corresponding apparatus for sterility testing allowing a rapid detection of micro-colonies and micro-organisms in a closed device without the risk of contamination through the environment and without the use of additional reagents.

The above object is achieved by the method according to claim 1. The dependent claims are directed to different advantageous aspects of the invention.

According to the invention there is provided a method for detecting micro-colonies growing on a membrane or an agarose medium of a sample in a closed device. The method comprising the steps:

irradiating the sample with a light incident at an angle β with respect to the normal to the membrane or the surface of the agarose medium from outside the closed device;

imaging an incident area of the light on the membrane or the surface of the agarose medium by means of a light receiving element using an imaging angle α different from angle β with respect to the normal to the membrane or the surface of the agarose medium from outside the closed device; detecting the light reflected or back scattered from the membrane or the surface of the agarose medium and/or the micro-colonies on the membrane and/or the micro-colonies on the agarose medium.

As a further aspect the above method is intended to use an electric powered light source, with a pattern illuminating the sample being a point, a line, a plurality of lines, a square pattern, an arc or circle pattern, a cross pattern, a multi-(square/round) pattern, a grid pattern or a two-dimensional area, a circle spot, a rectangular spot, or a square spot and wherein the light receiving element contains an array of pixel sensors.

As a further aspect the above method comprises the step of determining variations in the height of the incident area of the membrane or the surface of the agarose medium based on the position of the image of the incident light on the light receiving element using a triangulation method.

As a further aspect the above method is intended to detect contaminants like micro-colonies by determining a variation in the reflected, scattered and/or diffused light from the membrane or the surface of the agarose by using the number of pixels receiving a signal level above a threshold value around a light intensity peak for each line or column of an array of pixels of the pixel sensor.

As a further aspect the above method is intended to detect micro-colonies by determining a variation in the peak intensity value of the light reflected, scattered and/ or diffused from the membrane or the surface of the agarose by using the highest signal level of each line or column of an array of pixels of the pixel sensor.

As a further aspect the above method is intended to detect micro-colonies by determining a variation in the intensity value of the light reflected, scattered and/or diffused from the membrane or the surface of the agarose by using the signal level of the array of pixel sensor.

As a further aspect the above method comprises scanning the membrane or the surface of the agarose medium and/or the micro-colonies on the membrane and/or the micro-colonies on the agarose medium by a linear and/or rotational movement of the sample relative to the light receiving element.

As a further aspect of the above method the relative movement of the sample relative to the light receiving element is formed by a succession of a linear movement - displacing the incident area from the center of the sample towards the border thereof - followed by a rotational movement of the sample around a center axis thereof - displacing the incident area from the border of the sample towards the center thereof, or displacing the incident area from the center of the sample towards the border thereof - displacing the incident area from the border of the sample towards the center thereof - followed by a rotational movement of the sample around a center axis thereof.

As a further aspect the above method comprises arranging a light source and the light receiving element so as to illuminate and to image the entire membrane or the entire surface of the agarose medium while performing a relative motion of the sample in respect to the light source and the light receiving element.

As a further aspect in the above method an angle $\delta$ between the direction of the linear movement displacing the incident area from the center to the border and an imaging direction of the light receiving element is set to exceed an angle $\gamma$ between the direction of the linear movement displacing the incident area from the center to the border and the irradiation direction of the laser light.

As a further aspect in the above method the angle $\gamma$ between the direction of the linear movement displacing the incident area from the center to the border and the irradiation direction of the laser light is set to exceed the angle $\delta$ between the direction of the linear movement displacing the incident area from the center to the border and an imaging direction of the light receiving element.

Especially, the invention can make use of a 3D camera for the topography itself. Additionally the 2D imaging capability of the 3D camera can be used to provide additional information. For example a "quality map" (i.e. the highest grey value inside the line) recorded by the camera can be used at the same time as the topography. Alternatively or in addition, a "width map" (i.e. the line width) recorded by the camera can be used at the same time as the topography.

According to the invention it is possible to use solely height measurement or take benefit of a combination with other signals, like 2D picture, quality map, or width map.

In the following preferred embodiments of the invention will be described with reference to the accompanying drawings.

Fig. 1 is a schematic view of the arrangement of a laser light source and a 3D camera in relation to the sample;
Fig. 2 is a schematic view according to claim 1 when the incident area is close to the border of the sample;
Fig. 3 is a schematic view illustrating a scanning movement between the sample on the one hand and the light source and 3D camera on the other hand; and
Fig. 4 shows results of the scanning of a sample using the invention.

In the following, examples for carrying out the invention will be described with reference to the above-mentioried figures.

The invention is directed to a method for universally detecting micro-colonies growing on membranes (or an agarose medium) in a closed device, like the ones described in WO 2014/005669 A, by a topographic measurement implemented in order to avoid any "dead area" and "artifacts", and suitable for sterility testing application.

On one hand, any "dead area" is an increased risk of false negative results and would state the detection method as not being relevant for a sterility test application. On the other hand, "artifacts" would promote false positive rate and then would reduce the customer acceptance as the financial consequence would be the scrap of non-contaminated batches.

The topographic measurement is performed on usual membranes used for sterility application, i.e. mixed esters of celluloses membrane (HA membrane) and PolyVinyliDene Fluoride (PVDF or HV membrane) membranes for product with antibiotics or antimicrobial agents.

The topographic measurement uses the triangulation method with the help of a light, preferably LED light or laser light, and a camera. It avoids artifacts due to multi-reflexion of the light at the border of the closed device (vertical edges) and the light is always visible by the camera.

In a preferred embodiment a "LED Light" is used as a light source, since it prevents speckle effects which might be disturbing when using laser light sources. The LED Light can be arranged as an LED line.

The typical wavelength available with LED are near UV, i.e. 405 nm, blue, i.e. 465 nm, or green, i.e. 525 nm. However, the invention is not limited to these wavelengths.

First tests have shown that a wavelength of 465 nm produces less noise on HV membrane than other wavelengths.

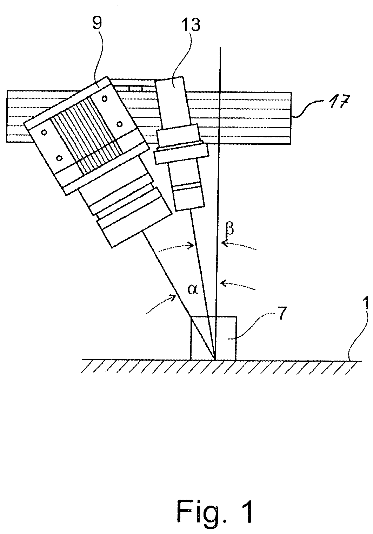

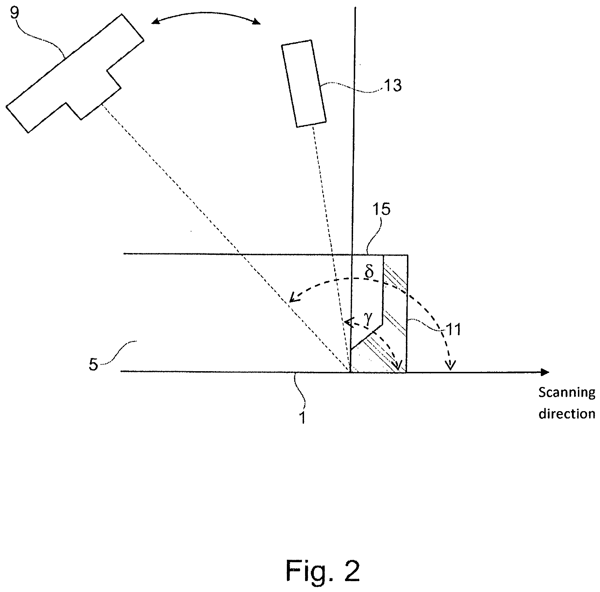

Figs. 1 and 2 show a membrane 1 which is provided in a closed device 3, comprising a transparent lid 15 forming together a sample 5. The sample 5 is irradiated with light from a light emitting device 13, for example a laser light source. The laser light source 13 irradiates the laser light towards the membrane 1 in the closed device 3 of the sample 5 through the transparent lid 15, so as to obtain an incident area 7 on the membrane.

A light receiving element 9, which in the embodiment is a camera (3D camera) comprising an array of pixel sensors, images the light incident area 7. The light receiving element 9 can be for example a CCD camera, CMOS camera, active pixel sensors, etc. The light incident area 7 can be any of a point, a line, one, two, three, etc. line pattern, square pattern, arc or circle pattern, cross pattern, Multi-(square/round) pattern, grid pattern or surface (circle spot, rectangular spot, square spot).

The laser light source 13 and the camera 9 are mounted together on a supporting structure 17 so as to have a defined and fixed positional relationship with respect to each other.

As an example, for the combination of laser light source 13 and light receiving element 9, respectively, it is possible to use a Keyence LJ-V 7060 line laser scanner having a wavelength of 405 nm and a CMOS as light receiving element with a sampling rate of 8 kHz. The length of a laser line i.e. the incident area 7, obtained by the scanner is around 15 mm. The scanner can be used at a distance of 60 mm from the membrane 1. The sample 5 will be placed on a motion table comprising a rotation table and a linear table, which might be provided with a gripper to grab the sample 5 and to place it on a stage.

According to the invention, the irradiation angle β and the imaging angle α, which are formed by the optical axis of the camera with regard to the normal on the surface of the membrane, or the irradiation direction of the laser light and the normal on the surface of the membrane, respectively, are preferably different from zero. Furthermore, these angles should be different from each other.

In one preferred embodiment α = 40° and β = 5°.

The values of α and β can be selected in a wide range. For example, the position of the laser light source 13 and the camera 9 can be inverted.

The camera 9 is connected with a micro-colony detecting unit (not shown), which will receive the image information obtained from the 3D camera and process this information to detect any micro-colonies. Therefore, by detecting the light reflected, scattered and/or diffused from the membrane or the surface of the agarose medium and/or the micro-colonies on the membrane and/or the micro-colonies on the agarose medium, and by processing and evaluating the position information of the detected reflected, scattered and/ or diffused light on the 3D camera it is possible to determine the presence of micro-colonies.

A micro-colony will have a certain growth in a direction perpendicular to the surface of the membrane. This can be detected.

According to the well-known equations of triangulation, a variation in the height dZ of the membrane 1 can be obtained by the following formula:

$$dZ = dX \, (\cos \beta) \, / \, (\sin (\alpha - \beta))$$

wherein dX is the variation of the position of the image of the incident area on the light-receiving element 9.

According to a preferred embodiment, the laser or LED light source 13 emits light so as to form a light incident area 7 in form of a line in Y direction on a flat portion of the membrane. Therefore, by performing a relative movement between the membrane 1 and the supporting structure 17 of laser light source 13 and camera 9 it is possible to scan a defined surface area of the membrane 1.

The sample 5 is prepared as follows. At first, a membrane 1 is used in a filtration process of a liquid in, for example, a pharmaceutical process. Thereafter, the sample is used for sterility testing by scanning the entire surface of the membrane without leaving any dead areas while maintaining the closed device 3 in a closed state in order to prevent contaminations at that stage. In an alternative embodiment of the present invention, after filtration and before sterility testing, the sample 5 is placed in an adequate environment to promote the growth of micro-colonies for a period of, for example, 5 days.

If the membrane 1 is located in the closed device 3, the border of the membrane is often immediately adjacent to the edge or rim 11 of the closed device 3. When scanning the sample it has to be ensured that this edge or rim 11 does not block neither the laser light nor the imaging of the light incident area 7 during the scanning procedure, so that no dead areas remain and so that no artefacts due to the edge or rim 11 occur.

Fig. 2 shows a case of approaching such an edge or rim 11 with the imaging system of Fig. 1. According to the invention, it is always ensured that the angle γ between the incident light and the actual scanning direction is larger than 90°. The same relation applies for the angle δ between the optical axis of the camera 9 and the scanning direction in case of approaching an edge or rim 11 of the closed device 3.

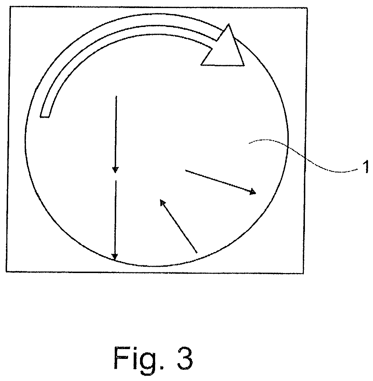

Fig. 3 shows an example of the preferred relative movements of the sample with regard to the light source 13 and the 3D camera 9 during scanning using the device shown in Figs. 1 or 2. In the preferred examples, the scanning sequence consists in scanning strips using a linear stage displacement of the sample 5. When the scan reaches the end of its strip, that is, when the scan reaches the rim or edge 11 of the closed device 3, the rotation stage rotates the sample 5 in order to place the laser on the next sector and start the scan to the next strip, and then moves the sample in the opposite direction, that is so as to displace the incident area 7 from the border of the closed device toward the center thereof.

The advantage of this special scanning sequence is that the form of the micro-colonies scanned will not be distorted or changed, since the scanning is always performed with a linear movement of the sample relative to the camera 9.

In order to reduce the overlap of strips, especially at the center of the sample 5, it is possible to start at a defined distance from the center with the linear movement towards the border of the sample 5. The distance depends on the width of the incident area. The remaining non-scanned area at the center of the membrane 1 should have a similar diameter to the width of a strip. Then, a first or a last scan at the center of the membrane covers this area. Thus, the whole membrane 1 will be scanned with this particular sequence as depicted in Fig.

3.

The number of scans depends on the width of each scan, respectively on the width of the incident area and the field of view of the camera. Due to the positioning errors of rotating tables, the minimum number of scanned stripes should be three or more.

A membrane scan is fast, e.g. a 47 mm membrane scan might take less than 1 min with at least a spatial resolution of 20 $\mu$m.

The usual signal to noise ratio allows to start detecting of micro-colonies at a size of 50 $\mu$m for a setup giving a spatial pitch of 25 $\mu$m according to the Nyquist-Shannon sampling theorem. This theorem states, with regard to the analysis of images, that the smallest detail to be resolved has to be sampled using at least two pixels. For micro-colonies presenting a low contrast regarding the membranes, for instance a white micro-colony on a white membrane, it means that the present invention is able to detect any micro-colony before the naked eyes. In fact, the topography measurement does not matter of color and contrast but only on the shape, i.e. height and diameter.

Typically, for most of germs, this means that they are detected within 5 or less days of incubation.

Traditional sample preparation for sterility testing with either HA or HV membranes can be performed as these membrane types are detected with laser wavelengths < 600 nm.

The present invention has the ability to detect all kind of germs, like *Methylobacterium extorquens, Propionibacterium acnes, Aspergillus brasiliensis, Dekkera anomala, Pseudomonas aeruginosa.*

As an alternative scanning sequence it is possible to rotate the sample when the incident area reaches the end of a linear movement towards the center of the sample.

In the above example the incident area had been described as a line. However, it is possible to use a plurality of lines or a different form for the incident area 7, as long as the form of the incident area 7 and the sequence of scanning movements ensure to completely scan the entire surface of the membrane 1 without leaving any dead areas.

Although the above example has been described with regard to a circular membrane 1, it is clear for a person skilled in the art that other forms of membranes 1 might be used, like rectangular membranes. The scanning sequence then should be adapted accordingly.

In order to perform the scanning through the closed lid 15 of the closed device 3, and have a light reflected from a transparent, semi-transparent or opaque membrane, the closed device 3 is arranged so as to have a determined distance between the lid 15 and the membrane 1, to have a light wavelength of < 600nm, for example 465 nm, and to perform a masking of the reflected, scattered and/or diffused light of the lid 15.

The "far" distance between the lid 15 of the closed device 3 and the membrane 1, allows to detect any micro-colony on either HA and HV membranes or other types of membrane though the transparent lid 15 even if these membranes become semi-transparent on an agar medium (liquid or solid). As mentioned, by placing the lid 15 at a "far" distance from the membrane, the emission of the laser line coming from the lid 15 will not hit the sensor of the camera 9 and will therefore not be detected.

If, for geometric reasons it is not possible to maintain the required far distance between the membrane 1 and the lid 15, it will be possible to set up the camera 9 in order to define a region of interest, which will contain the signal of the membrane 1, or stated otherwise, it will be possible to mask the signal from the lid 15 on the sensor.

Therefore, according to the invention, the camera 9 can be used for taking a grey scale two-dimensional picture of an area of interest in order to diagnose the cause of each artefact. In this regard, it should be taken care to provide a reasonable distance between the lid 15 and the membrane 1 in order to surely distinguish artefacts caused by the lid 15 or micro-colonies provided on the membrane 1. This would help to avoid false positive detections.

Although the above description has been given in connection with a HV membrane, other kinds of membranes 1, like HA membranes might be used as well. If these membranes 1 have a relatively flat surface, it is possible to start detecting micro-colonies at a size of around 50 $\mu$m for a set-up giving a spatial pitch of the detection device 25 $\mu$m.

Furthermore, for micro-colonies presenting a low contrast regarding the membranes, for example white micro-colonies on a white membrane, it means that it will be possible to detect these micro-colonies with the scanner before a naked eye detection would be possible.

The invention therefore allows to detect the micro-colonies with high reliability after an incubation time of typically 5 days or less.

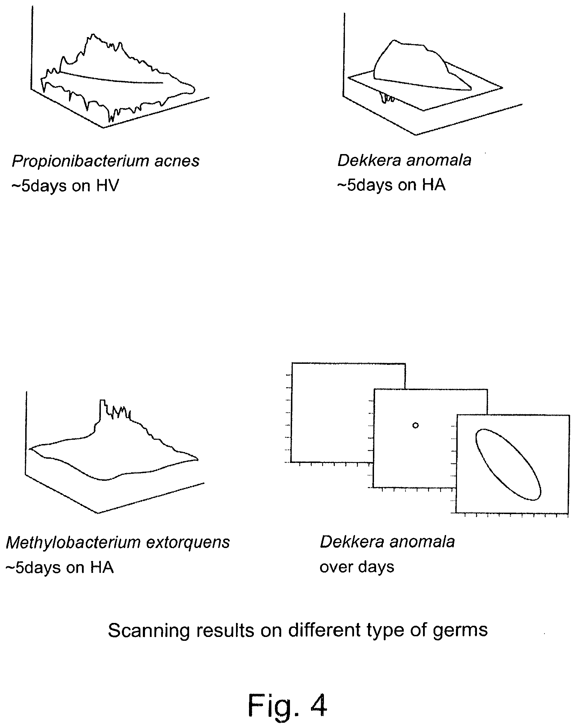

Examples of the topography given by some of micro-colonies on respective membranes 1 are depicted in Fig. 4.

From the result of the calculation of the topography, the areas of interest can be determined.

Additionally, two-dimensional images taken by the camera 9 of the areas of interest in a gray-scale or color form can be used to further distinguish between real areas of interest, where micro-colonies are growing on the membrane 1, and artefacts, which might be caused by dust, scratches or droplets at the lid 15.

Based on these steps, an evaluation of the sterility of the membrane can be done in a reliable and fast manner.

According to a second preferred embodiment in addition to the positional information of the reflected light on the camera used for the triangulation, additional image information obtained by the camera is involved in the determining of the presence or absence of any micro-colony.

According to this second embodiment the micro-colony detecting unit makes use of the color or grey scale image obtained by the camera 9, in order to distinguish more securely between artefacts and real micro-colonies. For example the detection of micro-colonies is performed by determining variation in the peak intensity value of the incident light on the membrane or the surface of the agarose or the micro-colonies on the membrane or the micro-colonies on the agarose medium by using the highest signal level for each column of the array of pixel sensor.

Alternatively the detection of contaminants is performed by determining variation in the intensity value of the incident light on the membrane or the surface of the agarose medium or the micro-colonies on the membrane or the micro-colonies on the agarose medium by using the signal level of the array of pixel sensor.

According to a third embodiment the variation in the form of the reflected light pattern might be used. For example the detection of micro-colonies is performed by determining variation in the width (e.g. thickness of the line) of the incident light on the membrane or the surface of the agarose medium or the micro-colonies on the membrane or the micro-colonies on the agarose medium by using the number of pixels that have a signal level above a given value around the light intensity peak for each column of the array of pixel sensor.

Of course the second and third example can be combined so as to make full use of the information of the image obtained by the camera.

Although the invention has been described based on a detailed examples, various modifications and variations might be implemented. In particular, the wavelength of the laser is not limited to 405 nm. Other laser light wavelengths, like 450, 465, 525, 532 nm might be used as well, although it is preferred that the laser light wavelengths should be below 600 nm.

These different wavelength can be used in connection with a LED-line as well.

Furthermore, the angle of the incident relate respectively to the normal of the membrane is set to 5° in the illustrated first embodiment. This value can, however, be larger or smaller as long as it is different from zero. In a similar way, the angle of the optical axis of the camera in the preferred first embodiment is set to 40°. This angle might have a different value also. Here again, it is important that the angle is different from zero with regard to the normal to the membrane surface. Furthermore, the two above angles should be different.

In the preferred first embodiment, the movement of the sample in the stage was a sequence of linear and rotational movements. This has the advantage that the form of a micro-colony detected during the linear movement will be imaged accurately, without distortions due to a rotational movement.

It is, however, possible to perform other kinds of movements, for example combined movements including linear displacement and rotation, as long as the image processing section of the apparatus is capable of reconstructing the actual form of the scanned area.

## Claims

1. Method for detecting micro-colonies growing on a membrane (1) of a sample (5) in a closed device or an agarose medium of a sample (5) in a closed device (3), the method comprising the steps:

   irradiating the sample (5) with a light incident at an angle ($\beta$) different from zero with respect to the normal to the membrane (1) or the surface of the agarose medium from outside the device (3);
   imaging an imaged incident area (7) of the light on the membrane (1) or the surface of the agarose medium by means of a light receiving element (9) using an imaging angle ($\alpha$) different from the light incident angle ($\beta$) with respect to the normal to the membrane (1) or the surface of the agarose medium from outside the device (3); the imaging angle ($\alpha$) being different from zero;
   detecting the light reflected, scattered and or diffused from the membrane or the surface of the agarose medium and/or the micro-colonies on the membrane and/or the micro-colonies on the agarose medium;
   the method being **characterized in that** it further comprises:

   scanning the membrane (1) or the surface of the agarose medium by a linear and rotational movement of the sample relative to the light receiving element (9); wherein the relative movement of the sample (5) relative to the light receiving element (9) comprises at least a succession of:

   a linear movement displacing the imaged incident area (7) from the center of the sample (5) towards the border thereof, followed by a rotational movement of the sample (5) around a center axis thereof, and a linear movement displacing the imaged incident area (7) from the border of the sample (5) towards the center thereof, or
   a linear movement displacing the imaged incident area (7) from the border of the sample (5) towards the center thereof, followed by a rotational movement of the sample (5) around a center axis thereof and a linear movement displacing the imaged incident area (7) from the center of the sample (5) towards the border thereof; and

wherein an angle $\gamma$ between the incident light and the direction of the linear movement displacing the imaged incident area (7) from the center of the sample (5) towards the border thereof and an angle $\delta$ between the direction of the linear movement displacing the imaged incident area (7) from the center of the sample (5) towards the border thereof and an imaging direction of the light receiving element (9) are set to exceed 90°.

2. Method according to claim 1, using an electric powered light source, with a pattern illuminating the sample, said pattern being a point, a line, a plurality of lines, a square pattern, an arc or circle pattern, a cross pattern, a multi-square/round pattern, a grid pattern or a two-dimensional area, a circle spot, a rectangular spot, or a square spot and wherein the light receiving element (9) contains an array of pixel sensors.

3. The method of claim 1 or 2, comprising the step of determining variations in the height of the imaged incident area (7) of the membrane (1) or the surface of the agarose medium based on the position of the image of the incident area on the light receiving element (9) using a triangulation method.

4. Method according to claim 1 or 2, wherein the detection of micro-colonies is performed by determining variation in the light by using the number of pixels receiving a signal level above a threshold value around a light intensity peak for each line or column of an array of pixels of the pixel sensor.

5. Method according to any of claims 1 to 4, wherein the detection of micro-colonies is performed by determining variation in the peak intensity value of the light by using the highest signal level for each line or column of the array of pixels of the pixel sensor.

6. Method according to any of claims 1 to 4, wherein the detection of micro-colonies is performed by determining variation in the intensity value of the light by using the signal level of the array of pixel sensor.

7. Method according to any of claims 1 to 6, comprising: performing the relative movement of the sample relative to the light source (13) and the light receiving element (9) so as to illuminate and to image the entire membrane (1) or the entire surface of the agarose medium.

8. Method according to claim 1, wherein the angle ($\delta$) is set to exceed an angle ($\gamma$).

9. Method according to claim 1, wherein the angle $\gamma$ is set to exceed the angle $\delta$.

**Patentansprüche**

1. Verfahren zur Detektion von Mikro-Kolonien, die auf einer Membran (1) einer Probe (5) in einer geschlossenen Vorrichtung oder einem Agarose-Medium einer Probe (5) in einer geschlossenen Vorrichtung (3) wachsen, wobei das Verfahren die Schritte umfasst:

Bestrahlen der Probe (5) mit einem Licht, das mit einem von Null verschiedenen Winkel ($\beta$) in Bezug auf die Normale zu der Membran (1) oder der Oberfläche des Agarose-Mediums von außerhalb der Vorrichtung (3) einfällt;
Abbilden eines abgebildeten Einfallsbereiches (7) des Lichtes auf der Membran (1) oder der Oberfläche des Agarose-Mediums mittels eines Lichtempfangselementes (9) unter Verwendung eines Aufnahmewinkels (a), der sich von dem Lichteinfallswinkel ($\beta$) unterscheidet, in Bezug auf die Normale der Membran (1) oder die Oberfläche des Agarose-Mediums von der Außenseite der Vorrichtung (3), wobei der Bildaufnahmewinkel ($\alpha$) unterschiedlich von 0 ist;
Erfassen des Lichts, das von der Membran oder der Oberfläche des Agarose-Mediums und/oder den Mikro-Kolonien auf der Membran und/oder den Mikro-Kolonien auf dem Agarose-Medium reflektiert, gestreut oder zerstreut wird;
wobei das Verfahren **dadurch gekennzeichnet ist, dass** es des Weiteren umfasst:

Abtasten der Membran (1) oder der Oberfläche des Agarose-Mediums durch eine lineare und Rotationsbewegung der Probe relativ zu dem Lichtempfangselement (9); wobei
die relative Bewegung der Probe (5) relativ zu dem Lichtempfangselement (9) zumindest eine Folge umfasst mit:

einer linearen Bewegung zur Verschiebung des abgebildeten Einfallsbereiches (7) von der Mitte der Probe (5) hin zu deren Rand, gefolgt durch eine Drehbewegung der Probe um deren Mittelachse und einer linearen Bewegung zur Verschiebung des abgebildeten Einfallsbereiches (7) von dem Rand der Probe (5) hin zu deren Mitte; oder einer linearen Bewegung zur Verschiebung des abgebildeten Einfallsbereichs (7) von dem Rand der Probe (5) hin zu deren Mitte, gefolgt durch eine Drehbewegung der Probe (5) um deren Mittelachse und einer linearen Bewegung zur Verschiebung des abgebildeten Einfallsbereichs (7) von der Mitte der Probe (5) hin zu deren Rand; und wobei ein Winkel (γ) zwischen dem Einfallslicht und der Richtung der linearen Bewegung zur Verschiebung des abgebildeten Einfallsbereichs (7) von der Mitte der Probe (5) hin zu deren Rand und einen Winkel (ठ) zwischen der Richtung der linearen Bewegung zur Verschiebung des abgebildeten Einfallsbereichs (7) von der Mitte der Probe (5) hin zu deren Rand und einer Bildrichtung des Lichtempfangselementes (9) eingestellt werden, um 90 Grad zu übersteigen,

2. Verfahren nach Anspruch 1 unter Verwendung einer elektrisch betriebenen Lichtquelle mit einem Muster, das die Probe beleuchtet, wobei das Muster ein Punkt, eine Linie, eine Mehrzahl von Linien, ein Quadratmuster, ein Bogen- oder Kreismuster, ein Kreuzmuster, ein Mehrfach-Quadrat-/Kreismuster, ein Gittermuster oder ein zweidimensionaler Bereich, ein kreisförmiger Spot, ein rechteckiger Spot oder ein quadratischer Spot ist und wobei das Lichtempfangselement (9) einen Array von Pixelsensoren umfasst.

3. Verfahren nach Anspruch 1 oder 2, mit dem Schritt des Bestimmens von Änderungen in der Höhe des abgebildeten Einfallsbereichs (7) oder der Membran (1) oder der Oberfläche des Agarose-Mediums beruhend auf der Position des Bildes des Einfallsbereiches auf dem Lichtempfangselement (9) unter Verwendung eines Triangulationsverfahrens.

4. Verfahren nach Anspruch 1 oder 2, bei dem die Erfassung der Mikro-Kolonien durch Bestimmen einer Änderung in dem Licht unter Verwendung der Anzahl von Pixeln durchgeführt wird, die ein Signalpegel oberhalb eines Schwellwertes empfangen, um einen Lichtintensitätsgipfel für jede Linie oder jede Spalte eines Arrays von Pixeln des Pixelsensors.

5. Verfahren nach einem der Ansprüche 1 bis 4, bei dem das Erfassen der Mikro-Kolonien durch Bestimmen der Änderung in dem Spitzenintensitätswert des Lichtes unter Verwendung des höchsten Signalpegels für jede Linie oder jede Spalte eines Arrays von Pixeln des Pixelsensors erfolgt.

6. Verfahren nach einem der Ansprüche 1 bis 4, bei dem das Erfassen der Mikro-Kolonien durch Bestimmen der Änderung des Intensitätswertes des Lichts unter Verwendung des Signalpegels des Arrays des Pixelsensors erfolgt.

7. Verfahren nach einem der Ansprüche 1 bis 6, mit: Durchführen der Relativbewegung der Probe relativ zu der Lichtquelle (13) und des Lichtempfangselementes (9), um so die gesamte Membran (1) oder die gesamte Oberfläche des Agarose-Mediums zu beleuchten und abzubilden.

8. Verfahren nach Anspruch 1, bei dem der Winkel (6) eingestellt wird, um größer als der Winkel (γ) zu sein.

9. Verfahren nach Anspruch 1, bei dem der Winkel (γ) eingestellt wird, um größer als der Winkel (ö) zu sein.

**Revendications**

1. Procédé pour détecter des micro-colonies croissant sur une membrane (1) d'un échantillon (5) dans un dispositif fermé ou sur un milieu d'agarose d'un échantillon (5) dans un dispositif fermé (3), le procédé comprenant les étapes suivantes :

irradiation de l'échantillon (5) avec une lumière incidente selon un angle (β) différent de zéro par rapport à la normale à la membrane (1) ou à la surface du milieu d'agarose à partir de l'extérieur du dispositif (3) ;
formation d'image d'une zone incidente imagée (7) de la lumière sur la membrane (1) ou la surface du milieu d'agarose au moyen d'un élément récepteur de lumière (9) par utilisation d'un angle de formation d'image (α) différent de l'angle de lumière incidente (β) par rapport à la normale à la membrane (1) ou à la surface du milieu d'agarose à partir de l'extérieur du dispositif (3) ; l'angle de formation d'image (α) étant différent de zéro ;
détection de la lumière réfléchie, dispersée et/ou diffusée à partir de la membrane ou de la surface du milieu d'agarose et/ou des micro-colonies sur la membrane et/ou des micro-colonies sur le milieu d'agarose ;
le procédé étant **caractérisé en ce qu'**il comprend en outre :

le balayage de la membrane (1) ou de la surface du milieu d'agarose par un mouvement linéaire et rotationnel de l'échantillon par rapport à l'élément récepteur de lumière (9) ; dans lequel
le mouvement relatif de l'échantillon (5) par rapport à l'élément récepteur de lumière (9) comprend au moins une succession de :

un mouvement linéaire déplaçant la zone incidente imagée (7) à partir du centre de l'échantillon (5) en direction de sa bordure, suivi d'un mouvement rotationnel de l'échantillon (5) autour d'un axe central de celui-ci, et d'un mouvement linéaire déplaçant la zone incidente imagée (7) à partir de la bordure de l'échantillon (5) en direction de son centre, ou

un mouvement linéaire déplaçant la zone incidente imagée (7) à partir de la bordure de l'échantillon (5) en direction de son centre, suivi d'un mouvement rotationnel de l'échantillon (5) autour d'un axe central de celui-ci et d'un mouvement linéaire déplaçant la zone incidente imagée (7) à partir du centre de l'échantillon (5) en direction de sa bordure ; et

dans lequel l'angle γ entre la lumière incidente et la direction du mouvement linéaire déplaçant la zone incidente imagée (7) à partir du centre de l'échantillon (5) en direction de sa bordure et l'angle δ entre la direction du mouvement linéaire déplaçant la zone incidente imagée (7) à partir du centre de l'échantillon (5) en direction de sa bordure et la direction de formation d'image de l'élément récepteur de lumière (9) sont établis de façon à dépasser 90°.

2. Procédé selon la revendication 1, utilisant une source de lumière à alimentation électrique, avec un motif éclairant l'échantillon, ledit motif étant un point, une ligne, une pluralité de lignes, un motif carré, un motif en arc ou en cercle, un motif en croix, un motif de carrés/ronds multiples, un motif en grille ou une zone bidimensionnelle, un point rond, un point rectangulaire, ou un point carré, et dans lequel l'élément récepteur de lumière (9) contient un réseau de capteurs de pixels.

3. Procédé selon la revendication 1 ou 2, comprenant l'étape de détermination de variations de la hauteur de la zone incidente imagée (7) de la membrane (1) ou de la surface du milieu d'agarose sur la base de la position de l'image de la zone incidente sur l'élément récepteur de lumière (9), par utilisation d'un procédé de triangulation.

4. Procédé selon la revendication 1 ou 2, dans lequel la détection de micro-colonies est effectuée par détermination de la variation de la lumière par utilisation du nombre de pixels recevant un niveau de signal supérieur à une valeur de seuil autour d'un pic d'intensité lumineuse pour chaque ligne ou colonne d'un réseau de pixels du capteur de pixels.

5. Procédé selon l'une quelconque des revendications 1 à 4, dans lequel la détection de micro-colonies est effectuée par détermination de la variation de la valeur d'intensité de pic de la lumière par utilisation du niveau de signal le plus élevé pour chaque ligne ou colonne du réseau de pixels du capteur de pixels.

6. Procédé selon l'une quelconque des revendications 1 à 4, dans lequel la détection de micro-colonies est effectuée par détermination de la variation de la valeur d'intensité de la lumière par utilisation du niveau de signal du réseau du capteur de pixels.

7. Procédé selon l'une quelconque des revendications 1 à 6, comprenant :
la réalisation du mouvement relatif de l'échantillon par rapport à la source de lumière (13) et à l'élément récepteur de lumière (9) de façon que toute la membrane (1) ou toute la surface du milieu d'agarose soit éclairée et imagée.

8. Procédé selon la revendication 1, dans lequel l'angle (δ) est établi de façon à dépasser l'angle (γ).

9. Procédé selon la revendication 1, dans lequel l'angle γ est établi de façon à dépasser l'angle δ.

Fig. 1

Fig. 2

Fig. 3

Propionibacterium acnes
~5days on HV

Dekkera anomala
~5days on HA

Methylobacterium extorquens
~5days on HA

Dekkera anomala
over days

Scanning results on different type of germs

# Fig. 4

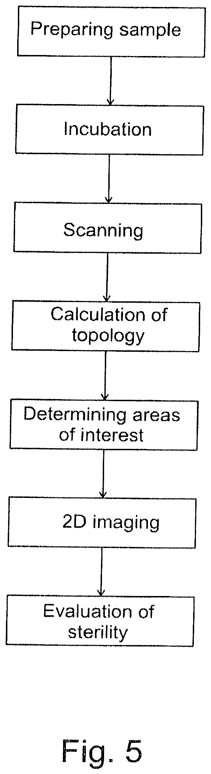

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011125033 A1
- WO 2011051481 W
- EP 1428018 B1
- WO 2014005669 A
- WO 2008137746 A1
- WO 3022999 A2
- WO 2014067907 A1
- WO 2012119114 A2
- WO 2012072467 A2

**Non-patent literature cited in the description**

- **MARCOUX PIERRE R et al.** Optical forward-scattering for identification of bacteria within microcolonies. *APPLIED MICROBIOLOGY AND BIOTECHNOLOGY,* 12 January 2014, 2043-2054
- **KIRSTIN PETERSEN et al.** Visual observation and analysis of animal and insect behaviour 2012. *3D Tracking of Building Processes in Macrotermes,* 11 November 2012, 1-4, http://people.seas.harvard.edu/~jkwerfel/vaib_icpr.pdf
- **PIN-TZU SU et al.** Bacterial Colony from Two-Dimensional Division to Three-Dimensional Development. *PLOS ONE,* 14 November 2012, vol. 7 (11